# EUROPEAN PATENT APPLICATION

(11) **EP 4 674 380 A1**
(43) Date of publication of application: **07.01.2026**
(21) Application number: 24382720.1
(22) Date of filing: 04.07.2024
(51) Int. Cl.: A61F 2/14

(54) **SUPPORT ELEMENT FOR AN ARTIFICIAL CORNEA**

(71) Applicant: Universitat Internacional De Catalunya, Fundació Privada, 08017 Barcelona (ES); Universitat Politècnica De Catalunya, 08034 Barcelona (ES)
(72) Inventor: GÓMEZ GONZÁLEZ, Silvia, 08034 Barcelona (ES); MANERO PLANELLA, Jose Maria, 08034 Barcelona (ES); BARRAQUER COMPTE, Rafael, 08017 Barcelona (ES); LAMARCA MATEU, José, 08017 Barcelona (ES); GIL MUR, Javier, 08017 Barcelona (ES)
(74) Representative: Torner, Juncosa I Associats, SL

(57) **Abstract**

The present disclosure relates to a support element (10) for an artificial cornea, the support element being disc-shaped and concentric with a central axis (P) and comprising: a central region (101) and a perimetral (102) region adjacent to a perimeter edge of the support element, the central region comprising a convex face (1011), a concave face (1012) opposed to each other and one central through hole (103) concentric with the central axis; wherein the perimetral region is flared, the surface of the support element is coated with silver, and at least the concave face of the support element comprises a peptide or a pharmaceutical salt thereof attached therein. The present disclosure also relates to an artificial cornea comprising the support element described.

## Description

### TECHNICAL FIELD

The present disclosure relates to a support element for an artificial cornea.

### BACKGROUND ART

The proper treatment of severe corneal damage is one of the greatest challenges in ophthalmology. It can arise from a variety of causes, but always ends with corneal blindness or scarring that is not treatable with common techniques such as partial corneal transplantation (ex. Descemet Stripping Automated Endothelial Keratoplasty "DSAEK" or Descemet's membrane endothelial keratoplasty "DMEK") or conventional corneal transplantation (penetrating keratoplasty). In these circumstances, an "artificial cornea" Keratoprosthesis (Kpro) forms the last resort for bilateral end-stage corneal blindness treatment.

For instance, US5354332A discloses an artificial cornea comprising a transparent optical part placed in an orifice formed in the cornea.

The most frequently and commonly performed Kpro are the Boston Type 1, the modified osteo-odonto Kpro (OOKP) and the osteo-Kpro with tibial bone.

Boston KPro has a collar button design, composed of a front plate with a stem, which houses the optical portion of the device and a back plate with or without a titanium locking c-ring. The back plate can be made of polymethylmethacrylate (PMMA) or titanium. Boston KPro is available in type I and type II formats. The type I design is used much more frequently than the type II which is reserved for severe end stage dry eye conditions and is similar to the type I except in that it has a 2 mm anterior nub designed to penetrate through a complete tarsorrhaphy.

During implantation, the device is assembled with a complete thickness donor corneal graft positioned between the front and back plate which is then sutured into place in a similar fashion to penetrating keratoplasty (corneal transplantation).

Most common postoperative complications in order of decreasing prevalence include retroprosthetic membrane (RPM), elevated intraocular pressure/glaucoma and infectious endophthalmitis.

OOKP (also known as "tooth in eye" surgery) consists of the removal of a tooth from the patient or a donor, after removal, a lamina of tissue cut from the tooth is drilled and the hole is fitted with an optic cylinder. The lamina is grown in the patients' cheek for a period of months and then is implanted upon the eye.

The most common postoperative complications in OOKP include extrusion, retroprosthetic membrane (RPM), elevated intraocular pressure/glaucoma, infection and retinitis.

Several solutions are available in the prior art, for instance US2020390539 A1 discloses an artificial cornea having an associated microstructure in the two sides thereof and being made, at least partially, of a hydrogel. The hydrogel may include bioactive molecules such as peptides.

A new keratoprosthesis is therefore needed which emulates the success of previous Kpro ideally reducing the resultant complications or defects. The ideal keratoprosthesis should have optimal bio-integration, resist infection, replicate qualities of the cornea including drug penetration and intraocular pressure measurements, reduce the risk of extrusion or rejection and last the lifetime of the patient.

### SUMMARY OF THE DISCLOSURE

The present disclosure relates, in a first aspect, to a support element for an artificial cornea, the support element being disc-shaped and concentric with a central axis and comprising a central region and a perimetral region adjacent to a perimeter edge of the support element, the central region comprising a convex face, a concave face opposed to each other and one central through hole concentric with the central axis; wherein the perimetral region is flared, the surface of the support element is coated with silver, and at least the concave face of the support element comprises a peptide or a pharmaceutical salt thereof attached therein.

The present disclosure also relates, in a second aspect, to an artificial cornea comprising the support element as disclosed herein.

The present disclosure also relates, in a third aspect, to a method to coat a support element for an artificial cornea.

### BRIEF DESCRIPTION OF THE FIGURES

The foregoing and other advantages and features will be more fully understood from the following detailed description of an embodiment with reference to the accompanying drawings, to be taken in an illustrative and non-limitative manner, in which:
FIG. 1 schematically depicts a plant view of an embodiment of the support herein disclosed.
FIG. 2 schematically depicts a section view of an embodiment of the support herein disclosed.
FIG. 3 shows Scanning Electron Microscopy (SEM) micrographies of the titanium surface of the support element (in grey) coated with silver particles (in white), at a constant pulse and in two different voltages: 5 V (FIG. 3A) and 10 V (FIG. 3B).
FIG. 4 shows optical coherence tomography (OCT) images of the eyes of rabbits used in an *in vivo* assay after euthanasia and enucleation.
FIG. 5 shows images of surgical microscope of the eyes in the control and treatment groups before and after the excision of the posterior segment of rabbits used in the *in vivo* assay.

### DESCRIPTION OF THE DISCLOSURE

All terms as used herein in this application, unless otherwise stated, shall be understood in their ordinary meaning as known in the art. Other more specific definitions for certain terms as used in the present application are as set forth below and are intended to apply uniformly through-out the specification and claims unless an otherwise expressly set out definition provides a broader definition.

As used herein, the term "pharmaceutically acceptable salt" refers to those salts which are, within the scope of sound medical judgment, suitable for use in contact with the tissues of humans and lower animals without undue toxicity, irritation, allergic response and the like, and are commensurate with a reasonable benefit/risk ratio. Pharmaceutically acceptable salts are well known in the art. Examples of pharmaceutically acceptable, nontoxic acid addition salts are salts of an amino group formed with inorganic acids such as hydrochloric acid, hydrobromic acid, phosphoric acid, sulfuric acid and perchloric acid or with organic acids such as acetic acid, trifluoroacetic acid, oxalic acid, maleic acid, tartaric acid, citric acid, succinic acid or malonic acid or by using other methods used in the art such as ion exchange. Other pharmaceutically acceptable salts include adipate, alginate, ascorbate, aspartate, benzenesulfonate, benzoate, bisulfate, borate, butyrate, camphorate, camphorsulfonate, citrate, cyclopentanepropionate, digluconate, dodecylsulfate, ethanesulfonate, formate, fumarate, glucoheptonate, glycerophosphate, gluconate, hemisulfate, heptanoate, hexanoate, hydroiodide, 2-hydroxy-ethanesulfonate, lactobionate, lactate, laurate, lauryl sulfate, malate, maleate, malonate, methanesulfonate, 2-naphthalenesulfonate, nicotinate, nitrate, oleate, oxalate, palmitate, pamoate, pectinate, persulfate, 3-phenylpropionate, phosphate, picrate, pivalate, propionate, stearate, succinate, sulfate, tartrate, thiocyanate, p-toluenesulfonate, undecanoate, valerate salts, and the like. Salts derived from appropriate bases include alkali metal, alkaline earth metal, and ammonium. Representative alkali or alkaline earth metal salts include sodium, lithium, potassium, calcium, magnesium, and the like. Further pharmaceutically acceptable salts include, when appropriate, nontoxic ammonium, quaternary ammonium, and amine cations formed using counterions such as halide, hydroxide, carboxylate, sulfate, phosphate, nitrate, lower alkyl sulfonate and aryl sulfonate.

The term (C₁ -C₁₀)alkyl refers to a saturated straight or branched alkyl chain having from 1 to 10 carbon atoms. Illustrative non-limitative examples are: methyl, ethyl, propyl, isopropyl, butyl, isobutyl, sec-butyl, tert-butyl, n-pentyl, neo-pentyl and n-hexyl.

The term "amino acid" refers to a molecule containing both an amino group and a carboxyl group. Unless otherwise explicitly stated, the amino acid can have L- or D-configuration. Amino acids can be classified by the side chain group. There are basically four different classes of amino acids determined by different side chains: (1) non-polar, (2) polar and neutral (uncharged polar), (3) acidic and polar, (4) basic and polar.

In certain embodiments, an amino acid is an alpha amino acid. Suitable amino acids include, without limitation, natural alpha-amino acids such as L-isomers of the 20 common naturally occurring alpha-amino acids: alanine, arginine, asparagine, aspartic acid, cysteine, glutamine, glutamic acid, glycine, histidine, isoleucine, leucine, lysine, methionine, phenylalanine, proline, serine, threonine, tryptophan, tyrosine, and valine; natural beta-amino acids (e.g., beta-alanine); and unnatural amino acids.

The present disclosure relates to a support element for an artificial cornea, the support element being disc-shaped and concentric with a central axis and comprising: a central region and a perimetral region adjacent to a perimeter edge of the support element, the central region comprising a convex face, a concave face opposed to each other and one central through hole concentric with the central axis; wherein the perimetral region is flared, the surface of the support element is coated with silver, and at least the concave face of the support element comprises a peptide or a pharmaceutical salt thereof attached therein.

The support element as disclosed herein is equivalent to the so-called back plate in an artificial cornea.

An Boston keratoprosthesis as disclosed herein is a specific type of artificial cornea that is surgically implanted to restore vision in patients with corneal blindness or severe corneal disease.

The perimetral region of the support element of the artificial cornea according to the disclosure is flared or has a bell-shape end. That is, a flared or bell-shaped end can be defined as a section of a solid or surface that transitions from a narrower to a wider cross-section in a smooth and continuous manner.

The support element may comprise a plurality of non-central through holes. These through holes might be used to allow for the secure placement of sutures. These sutures are used to anchor the artificial cornea to the eye during the surgical implantation.

The plurality of non-central through holes may be symmetrically distributed with respect to the central axis. The non-central through holes are preferably either eight or sixteen.

The non-central through holes may have a diameter lower than the diameter of the central through hole. This restriction accounts generally for a front optical cylinder that replaces the damaged or diseased cornea and is placed through the central through hole of the support disclosed.

The support element according to the disclosure is preferably made of titanium. Other alternatives are polymethylmethacrylate (PMMA) or similar biocompatible materials.

The support element might be submitted to a sandblasting procedure to obtain a rugous surface. An increased rugosity of the surface yields better performance of the prothesis, including, but not limited to, better cellular adhesion to the body tissues in the eye.

The silver coating in the support element as disclosed herein may comprise silver particles, in particular, silver nanoparticles. Silver is well known for its antibacterial capacity, and functionalizing the support of the artificial cornea, in particular the side that will be in contact with the live tissue, has special relevance. The amount of silver, though, has to be fined tuned to ensure an optimal performance in terms of retaining antibacterial properties but not generating cytotoxicity.

To obtain a support element with silver particles, the electrodeposition technique is favored. This technique allows stable and modified surfaces of i.e. titanium to be obtained. This also has the advantage of increasing the storage time of the support (backplates) without affecting their properties.

The peptide attached to the support element as disclosed herein may be an antifibrotic peptide. The antifibrotic peptide is preferably related to peptide P17, a molecule known to inhibit Transforming growth Factor beta (TGF- β).

The sequence of the antifibrotic peptide has at least an 85% sequence identity with SEQ ID NO:1: KRIWFIPRSSWY or SEQ ID NO:2: KRIWFIPRSSWYERA, or combinations thereof. The antifibrotic peptide may preferably be SEQ ID NO:1: KRIWFIPRSSWY or SEQ ID NO:2: KRIWFIPRSSWYERA, or combinations thereof.

In another embodiment according to the present disclosure, the peptide or salt thereof is a peptide which has an identity of 85%, 86%, 87%, 88%, 89%, 90%, 91 %, 92%, 93%, 94%, 95%, 96%, 97%, 98%, 99% or 100% with respect to SEQ ID NO: 1 or SEQ ID NO:2. In another embodiment, the peptide or salt thereof is a peptide which has an identity of 100% with respect to any of the sequence SEQ ID NO: 1 or SEQ ID NO:2.

In the present disclosure the term "identity" refers to the percentage of residues that are identical in the two sequences when the sequences are optimally aligned. If, in the optimal alignment, a position in a first sequence is occupied by the same amino acid residue as the corresponding position in the second sequence, the sequences exhibit identity with respect to that position. The level of identity between two sequences (or "percent sequence identity") is measured as a ratio of the number of identical positions shared by the sequences with respect to the size of the sequences (i.e., percent sequence identity = (number of identical positions/total number of positions) x 100).

A number of mathematical algorithms for rapidly obtaining the optimal alignment and calculating identity between two or more sequences are known and incorporated into a number of available software programs. Examples of such programs include the MATCHBOX, MULTAIN, GCG, FASTA, and ROBUST programs for amino acid sequence analysis, among others. Preferred software analysis programs include the ALIGN, CLUSTAL W, and BLAST programs (e.g., BLAST 2.1, BL2SEQ, and later versions thereof).

For amino acid sequence analysis, a weight matrix, such as the BLOSUM matrixes (e.g., the BLOSUM45, BLQSUM50, BLOSUM62, and BLQSUM80 matrixes), Gonnet matrixes, or PAM matrixes (e.g., the PAM30, PAM70, PAM120, PAM 160, PAM250, and PAM350 matrixes), are used in determining identity.

The BLAST programs provide analysis of at least two amino acid sequences, either by aligning a selected sequence against multiple sequences in a database (e.g., GenSeq), or, with BL2SEQ, between two selected sequences. BLAST programs are preferably modified by low complexity filtering programs such as the DUST or SEG programs, which are preferably integrated into the BLAST program operations. If gap existence costs (or gap scores) are used, the gap existence cost preferably is set between about -5 and -15. Similar gap parameters can be used with other programs as appropriate. The BLAST programs and principles underlying them are further described in, e.g., Altschul et al., "Basic local alignment search tool", 1990, J. Mol. Biol, v. 215, pages 403-410.

For multiple sequence analysis, the CLUSTAL W program can be used. The CLUSTAL W program desirably is run using "dynamic" (versus "fast") settings. Amino acid sequences are evaluated using a variable set of BLOSUM matrixes depending on the level of identity between the sequences. The CLUSTAL W program and underlying principles of operation are further described in, e.g., Higgins et al., "CLUSTAL V: improved software for multiple sequence alignment", 1992, CABIOS, 8(2), pages 189-191.

Moreover, some modifications might be introduced in the antifibrotic peptides, being for instance the addition of at least one moiety comprising 6-aminohexanoic acid (or 6-aminocaproic acid) as a linker and adding at least one moiety comprising L-3,4-dihydroxyphenylalanine (L-DOPA) as anchoring unit.

The antifibrotic peptide in the support element may preferably comprise a sequence having at least an 85% sequence identity with respect to SEQ ID NO:3: KRIWFIPRSSWY(X1)ₘ(X2)ₙ wherein m and n represent integers and are selected from 1, 2 or 3; C-terminal end corresponds to -C(O)R₁; and N-terminal end corresponds to -NHR₂ wherein X1 is 6-aminohexanoic acid, X2 is L-3,4-dihydroxyphenylalanine, R₁ is a radical selected from the group consisting of -OH and -NR₃R₄; R₂ is a radical selected from the group consisting of -H, -C(O)Rs; wherein R₃, R₄, R₅ are radicals independently selected from the group consisting of -H and (C₁-C₁₀)alkyl.

Preferably, in SEQ ID:NO:3: m and n are 2 and/or R₁ is -NR₃R₄ where R₃ and R₄ are -H and/or R₂ is -C(O)R₅ where R₅ is ethyl.

In another embodiment according to the present disclosure, the peptide or salt thereof is a peptide which has an identity of 85%, 86%, 87%, 88%, 89%, 90%, 91 %, 92%, 93%, 94%, 95%, 96%, 97%, 98%, 99% or 100% with respect to SEQ ID NO: 3. In another embodiment, the peptide or salt thereof is a peptide which has an identity of 100% with respect to any of the sequence SEQ ID NO: 3.

Preferably, the sequence of the antifibrotic peptide has at least an 85% sequence identity with SEQ ID NO:4: Ac-KRIWIFIPRSSWY-Acp-Acp-OOPA-OOPA-NH₂. Ac, stands for acetyl, Acp stands for 6-aminohexanoic acid (or 6-aminocaproic acid), DOPA stands for L-3,4-dihydroxyphenylalanine.

In another embodiment according to the present disclosure, the peptide or salt thereof is a peptide which has an identity of 85%, 86%, 87%, 88%, 89%, 90%, 91 %, 92%, 93%, 94%, 95%, 96%, 97%, 98%, 99% or 100% with respect to SEQ ID NO: 4. In another embodiment, the peptide or salt thereof is a peptide which has an identity of 100% with respect to any of the sequence SEQ ID NO: 4.

The support element according to the disclosure may also comprise an antifibrotic peptide that is attached to the support element by any of the hydroxyl groups in the X2 residue.

The present disclosure also relates to a to an artificial cornea comprising the support element as disclosed herein. The artificial cornea would comprise the support element and a frontal optical cylinder.

The present disclosure also relates, in a third aspect, to a method to coat a support element for an artificial cornea. The method comprises electrodepositing silver to the support element, as disclosed above, and coupling an antifibrotic peptide to the support element, in particular, coupling the antifibrotic peptide to the concave face of the support element.

The antifibrotic peptide might be obtained from available methods in peptide synthesis.

The present disclosure thus relates to a novel keratoprosthesis or artificial cornea comprising a front optical cylinder and a silver-coated and peptide-coated titanium support element having a flared end. The present invention reduces the risk of infection through the coating of the anterior and inner surface of the back plate with silver nanoparticles. It also reduces the risk and formation of retroprosthetic membranes through the coating of the posterior surface of the back plate with antifibrotic peptides and the flared end of the back plate which would prevent the retroprosthetic membrane from covering the optical part (cylinder) of the prosthesis. Finally, the rough surface of the areas of the back plate that are in contact with the cornea improve adhesion, reducing the risk of extrusion.

### DETAILED DESCRIPTION OF AN EMBODIMENT

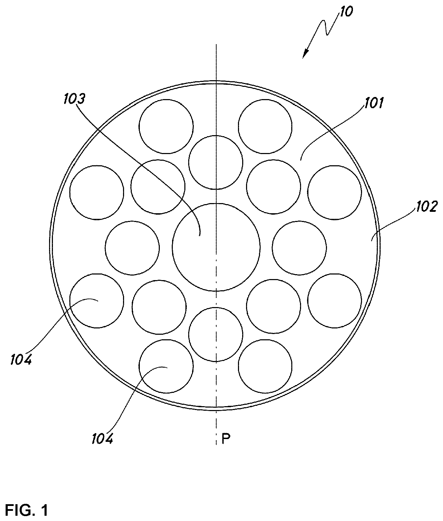

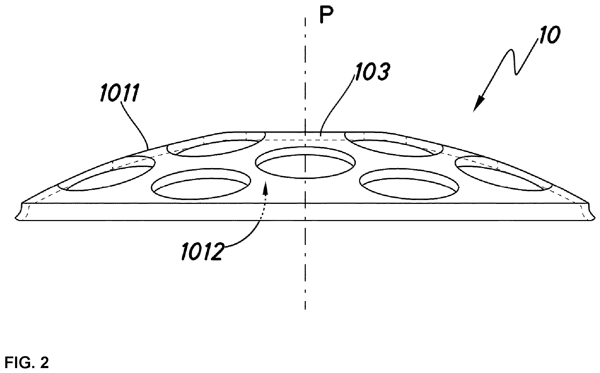

As disclosed above, FIGs. 1 and 2 show an embodiment of the support element (10) for an artificial cornea as herein disclosed. FIG. 1 and FIG. 2 show the same support element (10) from two perspectives, a plant view and a section view, respectively.

The support element (10) is disc-shaped and concentric with a central axis (P). The support element comprises a central region (101) and a perimetral region (102) adjacent to a perimeter edge of the support element (10). That is, the perimetral region (102) is the edge or border region of the support element (10). The central region (101) comprises a convex face (1011) and a concave face (1012) opposed to each other, and the central region (101) also comprises a one central through hole (103) concentric with the central axis (P). FIG. 2 illustrates the convex face (1011) and the concave face (1012) of the support element (10).

The support element (10) is characterized in that the perimetral region (102) is flared, bell-shape like, as shown in FIG. 2.

Moreover, the surface of the support element (10) is coated with silver and at least the concave face (1012) of the support element (10) comprises a peptide or a pharmaceutical salt thereof attached therein.

In FIG. 1 and FIG. 2, a plurality of non-central through holes (104) are also shown. In particular, the non-central through holes (104) in FIG. 1 and FIG. 2 are symmetrically distributed over the central axis (P). FIG. 1 shows a preferred embodiment wherein the support element (10) consists of 16 non-central through holes (104) symmetrically disposed over the central axis (P) and have a diameter that is lower than the central through hole (103).

### EXAMPLES

### Example 1: Silver coating of the support element

A support element for an artificial cornea (Boston Kerathoprosthesis) was provided. The material of the support element in this case was of Titanium (Ti).

The support element was introduced into an electrochemical cell to carry out the anodization process. A silver nitrate bath was used as a source of silver ions and sodium thiosulfate as a complexing agent. The backplate (or support element) was used as anode, and a platinum electrode was used as counter electrode.

Constant pulses of 5 to 10V and several cycles between 50 and 1000 was used.

Once the anodization was completed, the support element was washed with ethanol, distilled water and aceton for 15 minutes in an ultrasonic bath to remove all silver particles not solidly adhered to the titanium surface of the support element.

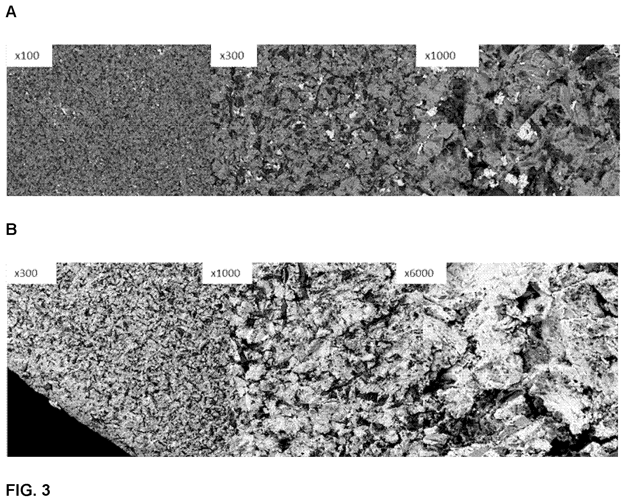

The surface of the support element was characterized. The presence of silver nanoparticles on the titanium surface was verified by scanning electron microscopy, as shown in FIG. 3, both when the constant pulses was 5 V (FIG. 3A) or 10 V (FIG. 3B).

### Example 2: Synthesis of an antifibrotic peptide

A derivative of P17 peptide was synthesized by solid phase peptide synthesis (SPPS) using Fmoc/tert-butyl chemistry. The target peptide was SEQ ID NO 4: Ac-KRIWIFIPRSSWY-Acp-Acp-DOPA-DOPA-NH₂. Acp stands for 6-aminohexanoic acid (or 6-aminocaproic acid), DOPA stands for L-3,4-dihydroxyphenylalanine. The resin used was a Rink Amide MBHA. After cleavage, a free amine group was obtained in the C-terminal. Acetylation was also carried out in the N-terminal.

The peptide was coupled to the concave side of the support element via the hydroxyl groups of DOPA.

The incorporation of the peptide to the backplate was confirmed by fluorescence microscopy when a fluorescent peptide analogue to the peptides herein described where coupled to the support element.

### Example 3: In vivo assays with rabbits

A proof-of-concept assay was performed using 15 rabbits that underwent eye surgery, 8 of them having conventional prosthesis implanted, while 7 others had the aritificial cornea comprising the support element as disclosed herein implanted.

Of the 15 rabbits that had undergone surgery, 3 (2 controls and 1 treated) were euthanized immediately after the surgery to serve as time 0 controls. During the follow-up of the rabbits, it was observed that all of them presented irritative eczema at the neck with hair loss, probably associated with the rubbing of the protective plastic and the humidity created by their water source. Without major complications and with good evolution until resolution in most cases.

No signs of active infection were observed in the eyes. Abundant secretions were not observed during the entire follow-up period, and the rabbits showed no objective signs of pain, loss of appetite, or other signs related to active infection.

Sustained bulbar and tarsal conjunctival hyperemia were observed, likely related to ocular surface exposure and the normal postoperative process. Additionally, stitches and prosthesis were maintained, except for specimen 06T, the treated sample in which complete extrusion occurred with extensive fibrin development after opening the tarsorrhaphy on day 30.

### Optical Coherence Tomography (OCT)

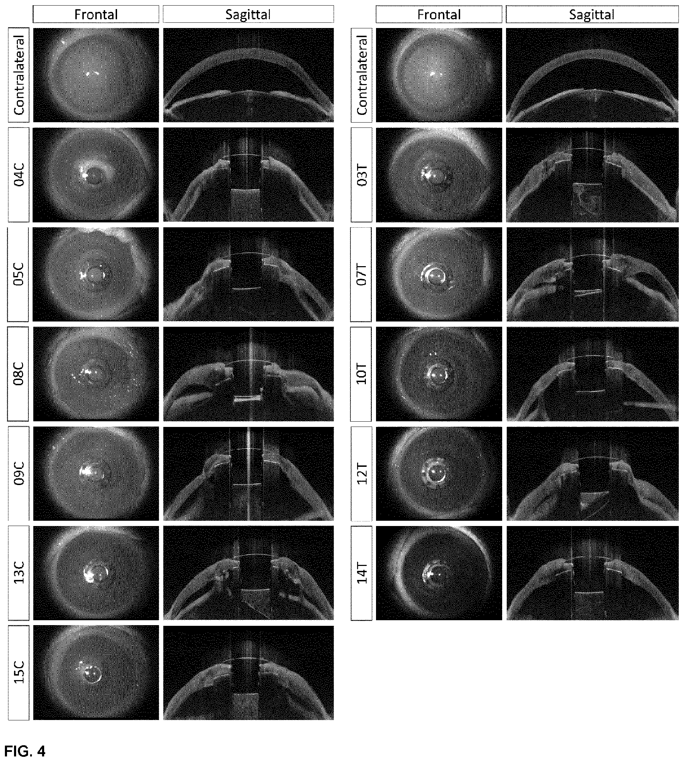

After enucleation, whole eye globe samples were analyzed using CASIA OCT. Images were taken in the frontal plane and sagittal plane (FIG. 4). The eye of the rabbit 06T, with total extrusion of the prosthesis, was excluded from this analysis. C refers to control group (implantation with unmodified artificial cornea) and T refers to treatment group (implantation of the artificial cornea comprising the support of the present disclosure).

Corneal melting was observed in 3 of the individuals in the control group (3 out of 6) and in 2 of the individuals in the treatment group (2 out of 5). Upon quantifying the sections with corneal melting, it was observed that in the control group, the area of corneal damage was approximately 37.5%, while in the treatment group, this area was 12.5%. In the control group, 5 out of 6 rabbits presented iris adhesion, whereas iris adhesion was observed in 4 out of 5 rabbits in the treatment group. A possible RPM behind the optic was identified in 2 out of 6 rabbits in the control group and in 2 out of 5 rabbits in the treatment group. However, OCT images were not used to definitively identify RPM, as the presence of the posterior capsule could lead to confusion and misclassification.

### Operation Microscope

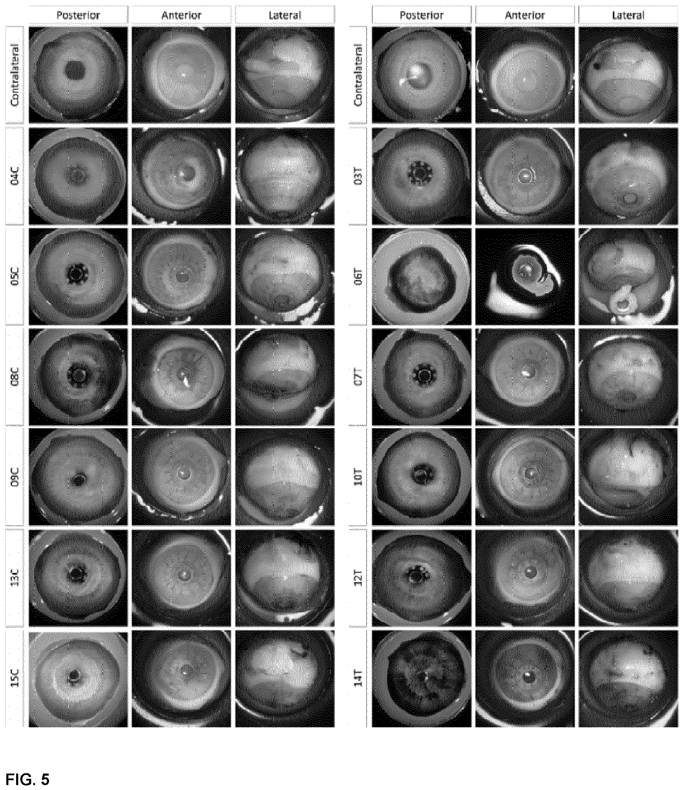

After the OCT measurements, the eyes were examined using a surgical microscope. Images were taken laterally and frontally of the complete eyeball and from the posterior view after excision of the anterior segment of the eyes (FIG. 5).

In the lateral view images, corneal ectasia was observed in the eye of rabbit 04C. Eye 05C showed a similar shape to the contralateral non-implanted eye. In the case of eye 08C, a loss of anterior chamber depth was observed, and corneal ectasia was also observed in rabbit 09C. Rabbits 13C and 15C showed a profile like that of a non-implanted eye. In the anterior view images, opacification was observed in eyes 04C, 05C, and 08C. Additionally, increased vascularization was observed in eye 08C, and eyes 09C and 13C showed a loss of transparency around the optic cylinder. Eye 15C exhibited greater loss of transparency, with opacification extending to the graft periphery. Retrocorneal membrane formation was observed in eyes 05C and 08C. In the posterior view, turbidity was observed in the aqueous humor of eye 04C, and iris adhesion to the corneal graft was confirmed to varying degrees in eyes 05C and 09C. Iris growth was observed in rabbit 09C, with adhesion at different points in 13C, and turbidity in the aqueous humor of rabbit 15C. Possible infection was noted in 2 out of 6 rabbits.

Surgical microscopic side view images of the treatment group samples showed maintenance of curvature in the case of rabbit 03T. For rabbit 06T, loss of the anterior chamber, clear loss of volume and turgor of the eye, and complete encapsulation of the prosthesis were observed. Eye 07T showed a slight loss of anterior chamber depth. Eyes 10T, 12T, and 14T exhibited a similar profile to the non-implanted eye. In the frontal view, a slight loss of transparency was observed around the optic cylinder in all eyes. An incipient retrocorneal membrane was observed around the stem in 03T and 07T. In the posterior view, iris adhesion was confirmed at different points in rabbits 03T and 07T.

In some cases, peripheral corneal neovascularization was observed, in the case of eye 8C, more marked at the superior level, and some cases with iridian neovascularization, in case 14T, probably secondary to a process of imbalance between anti-angiogenic factors and angiogenic factors due to inflammatory alteration, hypoxia and alteration of corneal innervation. With the risk of being the main risk factor for immune rejection of keratoplasty.

When analyzing the whole eye globe with an operation microscope with frontal illumination, signs of donor corneal graft opacification were visible in 4 of 5 rabbits in the treatment group, with an average graft opacification of 40%. In the control group, 5 of 6 rabbits presented graft opacification with an average opacification area of 60%. Opacification of the optical cylinder was found in one rabbit in the control group whereas it was also found in 3 of 6 rabbits in the treatment group.

Except for one control sample, this opacification did not correlate with the signs of retroprosthetic membrane seen in the OCT.

In the process of removing the residual lens, one treatment (07T) and 2 control samples (08C & 13C) had the capsule strongly adhered to the optic cylinder. The capsules in 07T and 13C were peeled away, whereas the capsule in 08C was strongly adhered and it was impossible to detach.

After separating the anterior segment from the rest of the eye globe, a certain degree of iris adhesion was observed in all the rabbits. The degree of adherence was slightly higher in control samples, averaging 85% (60 - 100%) of the iris circumference, whereas it was only 70% (30-100%) of the circumference in treated samples.

OCT studies therefore revealed fewer cases of corneal graft melting and reduced damage area in the treatment group. Corneal graft melting is a severe complication of keratoplasty. Moreover, the keratoplasty procedure itself is a risk factor that increases the likelihood of this complication. In the early stages, this complication is typically addressed pharmacologically; however, in advanced stages, it may be necessary to replace the prosthesis, and in the worst cases, the patient may lose the eye. In recent years, improvements in KPro design, coupled with advancements in surgical techniques, have contributed to a decrease in the incidence of corneal graft melting, although the exact incidence remains unclear due to the challenges in conducting large prospective studies. Several risk factors, such as infections, inflammation, or the materials used in the implant, are associated with this pathology. In this disclosure, the only difference between the groups was the use of anti-fibrosis and anti-infection coatings on the backplate. Therefore, the decrease in corneal graft melting could be attributed to these coatings.

Additionally, OCT revealed clusters behind the PMMA initially believed to be RPM; however, they were later identified as aggregates of fibronectin (FN). Since FN plays a crucial role in corneal stroma regeneration, it is commonly found on the surface of the grafts and recipient corneas after lamellar keratoplasty. Rabbits, in particular, are known to produce significant amounts of FN. Moreover, FN stimulates the production of fibrin, which also forms aggregates in rabbits. Although FN, fibrin, and other debris are usually cleared from the eye within a week, the adhesion of the iris to the cornea observed in some rabbits would have interfered with its removal from the anterior chamber.

Iridocorneal adhesion can decrease the iridocorneal angle, impeding proper drainage of the anterior chamber and leading to more severe complications such as glaucoma. Initially, OCT indicated iridocorneal adhesion in 5 out of 6 control rabbits and in 4 out of 5 treatment rabbits. However, subsequent surgical microscopy confirmed iridocorneal adhesion in all rabbits. The mean percentage of adherent iris was 70% in the treatment group and 80% in the control group. Although the difference is slight, a protective effect against iridocorneal adhesion was observed in individuals implanted with the dual backplate.

Surgical microscope observations revealed a reduced prevalence of corneal graft opacification and smaller opacification areas in the treatment group. Additionally, two rabbits in the control group were suspected of having infections (04C and 15C), and two others presented corneal ectasia (04C and 09C). Corneal ectasia is a deformation of the corneal tissue caused by factors such as infection or graft rejection. Corneal healing after an injury is a complex and highly regulated process in which the immune system plays a leading role. Proper control of inflammation and infection is essential to ensure adequate corneal healing, thereby preventing corneal graft opacification. In this disclosure, corneal graft opacification began around the PMMA optic and progressed towards the periphery of the graft, consistent with previous reports. This result aligns with previous findings indicating that PMMA is less compatible with corneal tissue and upregulates the expression of inflammatory cytokines, which can lead to secondary infections as corneal tissue melts around the PMMA. The absence of reported cases of corneal ectasia or infection in the treatment group, along with the lower incidence of corneal graft opacification, suggests that the applied coatings may have a synergistic effect that improves corneal healing.

In this disclosure, samples were observed by SEM to check for RPM. The RPM is a fibrovascular tissue that grows behind the backplate until it obstructs the optic. This tissue is generated as part of the host immune response and consists of distorted ECM components and fibroblasts and myofibroblasts. This membrane is thick and can be seen macroscopically behind the optic of affected patients. SEM observation of our samples revealed the presence of distorted ECM components, accompanied by the presence of fibrotic cells associated with the deposition of ECM components. The deposition of these components occurs 2 to 3 weeks after the corneal injury has occurred, coinciding with the completion of the differentiation process of myofibroblasts, which are primarily responsible for secreting ECM. So, in our observations, produced 4 weeks after keratoplasty, it is not surprising to find ECM deposition as part of the normal wound healing process. The deposition of ECM components is part of the natural corneal healing process and only becomes problematic when it is excessive, and its accumulation causes fibrosis and corneal malfunction. The lower incidence of presence of ECM components in the treatment group, as well as the lower presence of immune-like cells, suggests that the coatings may have reduced the inflammatory response.

In this disclosure the main comparison among groups was the safety assessment of the applied coatings. Even though the antimicrobial effect of silver deposits was not tested itself, the potential harmful effects on the surrounding tissue were discarded since the area of corneal graft melting was lower in the treatment group. Regarding the antifibrotic treatment, less fiber cell membrane formation and iridocorneal adhesion were observed in the treatment group. Finally, the flared end did not effectively prevent retroprosthetic membrane contact with the optic cylinder. This may be attributed to the presence of a 'slit' on the titanium back plate, which allows for it to snap onto the optic cylinder. This opening serves as a potential entry point for cells to grow on the optic cylinder, as observed in SEM analysis of samples 03T and 04C. Therefore, a design that eliminates this 'slit' would be more desirable.

The results herein disclosed show the possibility of modifying the backplate in a side-specific manner to avoid the usual complications of KPro. In this way, device retention would be improved without increasing pharmacological treatment.

As a summary, the combination of treating the support element of an artificial cornea with silver particles, a peptide and the support element having a flared end is possible, as characterized physicochemically, is safe, and involves and improvement in performance. The artificial cornea comprising the support element herein disclosed presented fewer infections, reduced deposition of ECM components, less corneal graft melting, opacification, and protection against iridocorneal adhesion. The observed absence of immune-like cells in the support element (or back plate) suggests that the applied coatings may effectively reduce the inflammatory response, thus decreasing the probability of RPM development. This finding is particularly promising as it addresses one of the key challenges in KPro surgery. This disclosure underscores the potential clinical benefits of modifying the backplate in a side-specific manner to mitigate common complications associated with KPro surgery. By enhancing device retention without necessitating additional pharmacological treatment, this approach offers a promising alternative for improving patient outcomes.

## Claims

1. Support element (10) for an artificial cornea, the support element (10) being disc-shaped and concentric with a central axis (P) and comprising:
a central region (101) and a perimetral region (102) adjacent to a perimeter edge of the support element (10),
the central region comprising a convex face (1011), a concave face (1012) opposed to each other and one central through hole (103) concentric with the central axis (P);
wherein
the perimetral region (102) is flared,
the surface of the support element (10) is coated with silver, and
at least the concave face (1012) of the support element (10) comprises a peptide or a pharmaceutical salt thereof attached therein.

2. The support element (10) according to claim 1, wherein the support element (10) comprises a plurality of non-central through holes (104).

3. The support element (10) according to claims 1 or 2, wherein the plurality of non-central through-holes (104) are symmetrically distributed with respect to the central axis (P).

4. The support element (10) according to claims 2 or 3, wherein the plurality of non-central through holes (104) are eight or sixteen.

5. The support element (10) according to any one of claims 1 to 4, wherein the non-central through holes (104) have a diameter lower than the diameter of the central through hole (103).

6. The support element (10) according to any one of the previous claims, wherein the support element (10) is made of titanium.

7. The support element (10) according to any one of the previous claims, wherein the support element (10) has been submitted to a sandblasting procedure to obtain a rugous surface.

8. The support element (10) according to any one of the previous claims, wherein the silver coating comprises silver nanoparticles.

9. The support element (10) according to claim 8, wherein the silver nanoparticles coating in the support (10) is obtained by means of electrodeposition.

10. The support element (10) according to any one of the previous claims, wherein the peptide is an antifibrotic peptide.

11. The support element (10) according to any one of the previous claims, wherein the antifibrotic peptide comprises a sequence having at least an 85% sequence identity with respect to SEQ ID NO:1: KRIWFIPRSSWY or SEQ ID NO:2: KRIWFIPRSSWYERA.

12. The support element (10) according to claim 10, wherein the antifibrotic peptide comprises a sequence having at least an 85% sequence identity with respect to SEQ ID NO:3: KRIWFIPRSSWY(X1)ₘ(X2)ₙ
wherein
- m and n represent integers and are selected from 1, 2 or 3;
- C-terminal end corresponds to -C(O)R₁; and
- N-terminal end corresponds to -NHR₂
wherein
X1 is 6-aminohexanoic acid
X2 is L-3,4-dihydroxyphenylalanine
R₁ is a radical selected from the group consisting of -OH and -NR₃R₄;
R₂ is a radical selected from the group consisting of -H, -C(O)Rs;
wherein
R₃, R₄, R₅ are radicals independently selected from the group consisting of: -H and (C₁-C₁₀)alkyl.

13. The support element (10) according to claim 12, wherein m and n are 2 and/or R₁ is - NR₃R₄ where R₃ and R₄ are -H and/or R₂ is -C(O)Rs where R₅ is ethyl.

14. The support element (10) according to claims 12 or 13, wherein the antifibrotic peptide is attached to the support element (10) by any of the hydroxyl groups in the X2 residue.

15. An artificial cornea comprising the support element (10) defined in claims 1 to 14.
